(19) Europäisches Patentamt
European Patent Office
Office européen des brevets

(11) Numéro de publication: **0 286 495 B1**

(12) **FASCICULE DE BREVET EUROPEEN**

(45) Date de publication de fascicule du brevet: **12.05.93** (51) Int. Cl.[5]: **C07D 413/12**, A61K 31/535

(21) Numéro de dépôt: **88400693.3**

(22) Date de dépôt: **23.03.88**

# (54) Dérivés de la morpholine, leur procédé de préparation et les compositions pharmaceutiques les renfermant.

(30) Priorité: **24.03.87 FR 8704047**

(43) Date de publication de la demande:
**12.10.88 Bulletin 88/41**

(45) Mention de la délivrance du brevet:
**12.05.93 Bulletin 93/19**

(84) Etats contractants désignés:
**AT BE CH DE FR GB IT LI LU NL SE**

(56) Documents cités:
**FR-A- 2 073 365**

(73) Titulaire: **ADIR ET COMPAGNIE**
**1 rue Carle Hébert**
**F-92415 Courbevoie Cédex(FR)**

(72) Inventeur: **Regnier, Gilbert**
**27 avenue du Plessis**
**F-92290 Chatenay Malabry(FR)**
Inventeur: **Guillonneau, Claude**
**21 rue des Bergers**
**F-92140 Clamart(FR)**
Inventeur: **Lepagnol, Jean**
**5 rue de Vlaminck**
**F-78400 Chatou(FR)**

(74) Mandataire: **Reverbori, Marcelle**
**Adir et Compagnie, 1, rue Carle Hébert**
**F-92415 Courbevoie Cédex (FR)**

EP 0 286 495 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés de la morpholine, leur procédé de préparation et les compositions pharmaceutiques les renfermant.

Elle concerne particulièrement
les dérivés de la morpholine de formule générale I :

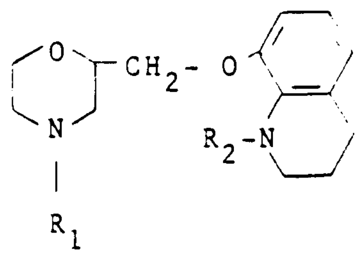

(I)

dans laquelle
$R_1$ représente :

- un atome d'hydrogène ;
- un radical alkyle, contenant de 1 à 6 atomes de carbone, en chaîne droite ou ramifiée, comportant éventuellement une double liaison ;
- un radical aralkyle de formule générale :

  $Ar-(CH_2)_m-$

  dans laquelle
  Ar représente un radical aryle éventuellement substitué par un ou plusieurs atomes d'halogène ou radicaux alkyle ou alkoxy contenant contenant chacun de 1 à 5 atomes de carbone, ou radicaux de formule $-O-(CH_2)_n-O-$ dans laquelle n prend les valeurs 1 ou 2 ; et
  m représente un nombre entier de 1 à 3 ;
- un radical cycloalkyle contenant 5 ou 6 atomes de carbone ; ou
- un radical acyle de formule : $R'-CO-$ dans laquelle R' représente un radical alkyle renfermant 1 ou 2 atomes de carbone ; et

$R_2$ représente :
un atome d'hydrogène ;
un radical alkyle, contenant de 1 à 6 atomes de carbone, en chaîne droite ou ramifiée, comportant éventuellement une double liaison ;
un radical aralkyle de formule générale : $Ar-(CH_2)_m-$ dans laquelle Ar et m sont tels que précédemment définis;ou
un radical acyle de formule $R'-CO-$ dans laquelle R' prend la signification énoncée précédemment ; et
leurs énantiomères.

Les composés de l'état antérieur de la technique les plus proches des dérivés (I) répondent à la formule générale

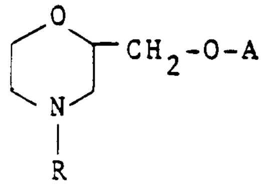

dans laquelle A représente :
un radical éthoxy-2 phényle, (cf. brevet belge n° 708557, dont le produit leader est la viloxazine) ;
un radical (thiényl-2 méthyl)-2 phényle, (cf. brevet britannique n° 1.466.820, dont le produit leader est la sufoxazine) ;

un radical indényle (cf. brevet ouest – allemand n° 2.601.703, dont le produit leader est l'indéloxazine) ;
un radical benzofurannyle, benzothiényle ou indolyle, (cf. brevet ouest – allemand n° 2.056.592, et Kokai japonais n° 75 – 129575) ;
un radical quinolone – 4 – yle (cf. Kokai japonais n° 77 – 116482).

Les dérivés de la présente invention se différencient de ceux de l'Art antérieur, non seulement par leur structure chimique mais aussi par leur comportement pharmacologique. Ils présentent en effet une activité anti – ischémique et anti – hypoxie supérieure à celle des dérivés de l'art antérieur précédemment cités sans en manifester les effets secondaires. En particulier, contrairement aux dérivés proches de l'Art antérieur, ils n'inhibent pas la recapture de la sérotonine. Ils sont psycho éveillants, ce que ne sont pas les produits les plus proches de l'Art antérieur, comme le prouvent les tests sur les narcoses. C'est ainsi que l'on a pu observer, avec les dérivés de la présente invention, une diminution de la narcose au barbital alors que la viloxazine par exemple reste sans effet sur cette narcose.

La présente invention a également pour objet le procédé de préparation des dérivés de formule générale I, caractérisé en ce que :
l'on fait réagir une morpholine substituée de formule générale II :

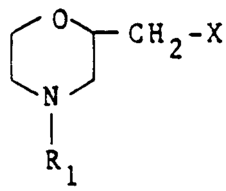

(II)

dans laquelle
$R_1$ prend la signification énoncée précedemment, et
X représente un atome d'halogène ou un radical tosyloxy,
avec l'hydroxyquinoléine de formule III :

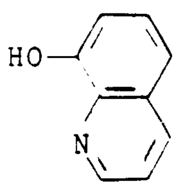

(III)

préalablement transformée en sel alcalin,
pour obtenir un composé de formule générale IV :

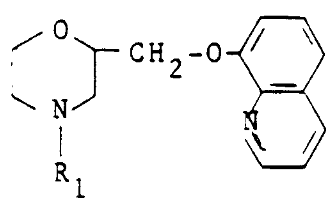

(IV)

dans laquelle $R_1$ a la signification énoncée précédemment, que l'on hydrogène ensuite pour obtenir un dérivé de formule Ia :

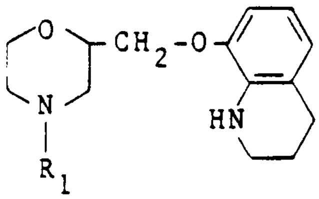

(Ia)

dans laquelle $R_1$ est tel que précédemment défini, lequel est alors condensé avec un dérivé de formule générale V :

$R'_2-X'$ (V)

dans laquelle :
$R'_2$ représente
un radical alkyle contenant de 1 à 6 atomes de carbone, en chaîne droite ou ramifiée, comportant éventuellement une double liaison ;
un radical aralkyle de formule générale : $Ar-(CH_2)_m-$,dans lequel Ar et m sont tels que précédemment définis et simultanément X' représente un atome de brome ou d'iode ou un radical tosyloxy ; ou
$R'_2$ représente un radical acyle de formule $R'-CO-$ dans laquelle R' a la signification énoncée précédemment, et
simultanément X' représente un atome de chlore ;
pour obtenir un dérivé de formule générale Ib :

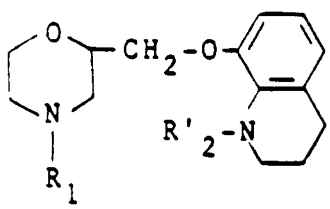

(Ib)

dans laquelle $R_1$ et $R'_2$ ont les significations énoncées précédemment.

L'ensemble des dérivés (Ia) et (Ib) forme l'ensemble des dérivés (I).

La réaction des dérivés (II) et (III) est réalisée de façon particulièrement adéquate en opérant dans un solvant choisi parmi les amides tertiaires tel que par exemple le diméthylformamide ou le diméthylacétamide, à une température comprise entre 90 et 110 °C en présence d'un accepteur du dérivé HX formé ; cet accepteur étant un agent alcalin dont le plus communément employé est l'hydrure de sodium en suspension dans l'huile à 50%.

On peut également, si on le désire, préparer les sels alcalins de l'hydroxyquinoléine par évaporation d'une solution hydroéthanolique de celle-ci et d'une base forte telle que l'hydroxyde de potassium ou de sodium, en quantité équimoléculaire.

L'hydrogénation du composé (IV) s'effectue à basse pression en présence d'un catalyseur appartenant aux métaux du VIIIème groupe tels que rhodium ou palladium (ce dernier étant préférentiellement sous forme d'hydroxyde) dans un solvant tels que les alcools de bas poids moléculaire , miscibles à l'eau comme le méthanol ou l'éthanol. Il est particulièrement recommandé d'opérer à une température comprise entre 20 et 60 °C, sous une pression d'hydrogène de $1.10^5$ à $6.10^5$ Pa en présence d'un catalyseur tel que Rh/C ou $Pd(OH)_2/C$. La condensation des dérivés (Ia) et (V) pour préparer les dérivés (Ib) est effectuée de manière particulièrement adéquate dans un solvant qui selon la valeur de $R'_2$ peut être un carbure aromatique, le tétrahydrofuranne ou un solvant polaire tels que les alcools à bas poids moléculaire, l'acétonitrile ou le diméthylformamide. Il est recommandé d'opérer à une température comprise entre 20 et 80 °C en présence d'un accepteur du composé HX' formé. Cet accepteur peut être une base tertiaire telle que par exemple, la triéthylamine, la pyridine ou la diméthylaminopyridine.

Ce schéma de synthèse s'applique particulièrement bien pour la préparation des énantiomères des composés (I) à partir d'une morpholine de formule II, dans laquelle X est un radical tosyloxy, dont les formes R et S peuvent être préparées selon la méthode de R. HOWE et coll. J. Med. Chem. (1976), 19,

1674 – 1676.

Les dérivés de formule (I) dans laquelle $R_1$ représente un atome d'hydrogène, donc répondant plus précisément à la formule générale I' :

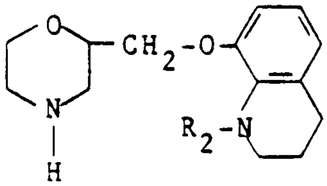

(I')

dans laquelle $R_2$ a la même signification que dans la formule I sont préférentiellement préparés à partir d'une morpholine de formule générale II'

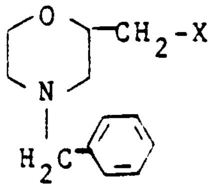

(II')

qui, condensée avec une hydroxyquinoléine (III) donne un dérivé de formule IV':

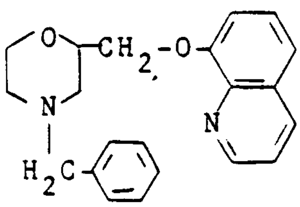

(IV')

lequel est soumis à une hydrogénation catalytique sous basse pression
(en opérant de préférence sous une pression de $2.10^5$ à $5.10^5$ Pa, avec un catalyseur au rhodium) pour obtenir un dérivé de formule IV"

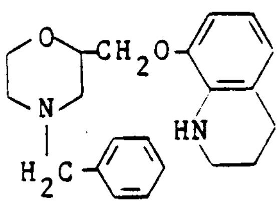

(IV")

lequel est :
soit débenzylé directement au moyen d'une hydrogénolyse à des pressions comprises entre $2.10^5$ et $5.10^5$ Pa. pour donner le dérivé de formule I'a

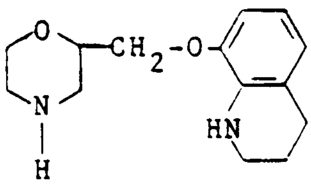

(I'a)

soit d'abord alkylé ou acylé au moyen de $R'_2X'$ tel que précédemment défini, puis débenzylé par hydrogénation catalytique sous basse pression (en opérant de préférence entre $2.10^5$ et $5.10^5$ Pa, en présence d'un catalyseur au palladium) pour obtenir le dérivé de formule I'b

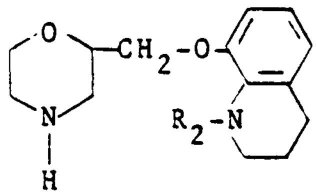

(I'b)

L'ensemble des dérivés (I'a) et I'b) représente l'ensemble des dérivés (I').

Cette deuxième méthode est également intéressante pour la préparation des dérivés de formule générale I dans laquelle $R_1$ et $R_2$ sont différents et en particulier lorsque $R_1$ est un radical comportant une double liaison, sensible aux conditions d'hydrogénation.

Enfin cette deuxième méthode permet également de préparer les dérivés de formule générale I dans laquelle $R_1$ et $R_2$ sont identiques et autres qu'hydrogène, par alkylation ou acylation du dérivé de formule I'a :

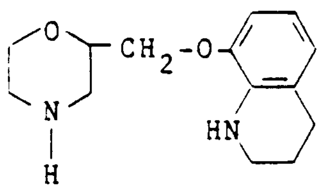

(I'a)

selon le procédé décrit précédemment.

Toutes ces variantes de préparation sont incluses dans la présente invention.

Les dérivés de la présente invention peuvent être purifiés par chromatoflash sur support $SiO_2$ (35 – 70µ) et séparés dans des systèmes tels que $CH_2Cl_2 - CH_3OH$, benzène – $CH_3OH$ sous des pressions d'azote allant de $0{,}5.10^5$ à $1.10^5$ Pa.

Ces dérivés donnent des sels avec les acides physiologiquement tolérables et se présentent le plus souvent, tant sous forme de base que de sel, à l'état amorphe. Ces sels sont également inclus dans la présente invention.

Les dérivés de la présente invention possèdent des propriétés pharmacologiques et thérapeutiques intéressantes. Ils s'opposent aux désordres de la fonction cérébrale générés par un déficit circulatoire ou par une baisse d'oxygénation, qui sont deux des composantes pathologiques étroitement associées à l'accident vasculaire cérébral et au vieillissement. Ces propriétés se manifestent grâce à un mécanisme d'action visant au maintien de la neurotransmission catécholaminergique dont le dysfonctionnement a également été largement démontré et impliqué dans les phénomènes dépressifs et amnésiques associés à ces pathologies cérébrales. Ces propriétés s'exercent de manière plus intense, plus spécifique et avec moins d'effets secondaires que le plus récent composé de référence anti – ischémique et anti – gériatrique agissant par amélioration de la biodisponibilité intrasynaptique en catécholamines : le bifemelane.

Les composés de la présente invention sont testés par leur capacité à prolonger la survie cérébrale de souris soumises à un arrêt circulatoire aigu par injection intraveineuse de chlorure de magnésium. Administrés par voie intrapéritonéale, les composés de l'invention augmentent très significativement le temps de survie dès la dose de 3mg/kg , alors que dans les mêmes conditions le bifemelane exerce un effet 3 à 5 fois moindre.

De la même façon, les composés de l'invention exercent leurs effets protecteurs cérébraux chez la souris soumise à une hypoxie globale aiguë (dépression barométrique à fraction inspirée en oxygène = 3,3). Cette protection est objectivée par l'augmentation du temps de survie du cerveau. L'effet antihydroxique est très significatif dès la dose de 3mg/kg aussi bien par voie IP (où l'on note une augmentation du temps de survie jusqu'à 30%) que par voie PO (où cet accroissement atteint 20%) ; ce qui indique la très bonne biodisponsibilité digestive des dérivés testés (notamment des produits objet des exemples 2 et 7a). Le bifemelane, administré dans les mêmes conditions n'exerce un faible effet protecteur (20%) qu'à la dose de 10mg/kg par voie IP et 30mg/kg par voie orale. A la dose de 30mg/kg per os, on a

observé une augmentation du temps de survie respectivement de 62 et 52% pour les produits des exemples 2 et 7a, une augmentation du temps de survie de 20% pour le bifemelane et de 32% pour la viloxazine.

L'examen des relations doses-effets vis-à-vis de cette même épreuve d'hypoxie cérébrale permet de constater, avec des doses croissantes de composés de l'invention, une parfaite progression de l'intensité de la protection sans aucune manifestation neurologique des animaux après l'administration du composé à tester.

Par exemple, aux doses orales de 10, 30, 50 et 100mg/kg le produit de l'exemple 7a exerce un effet protecteur de 29, 52, 73 et 118% respectivement.

Dans le cas du bifemelane administré par voie orale, cette linéarité n'est pas observée et l'effet protecteur (20% à 30mg/kg ; 68% à 100mg/kg ; 179% à 300mg/kg) est dû à l'apparition d'effets secondaires (apathie, somnolence) puis d'effets toxiques propres.

Sur ce test, l'index thérapeutique ($DL/_{50}/DE_{50}$) est très en faveur des composés de l'invention puisqu'à une activité protectrice 3 à 5 fois plus importante, s'associe, notamment pour la voie IP, une toxicité 2,5 fois plus faible (La $DL/_{50}$ étant de 270mg/kg pour les composés des exemples 2 et 7a par exemple, alors qu'elle est de 110mg/kg pour le bifemelane).

D'autre part, les composés de la présente invention ont été testés pour leurs capacités à faciliter la neurotransmission noradrénergique. Ainsi, chez la souris, il a été montré que ces composés induisent une mortalité chez des animaux recevant par voie IP une dose non léthale de yohimbine (30mg/kg). Par exemple, à la dose orale de 100mg/kg, la mortalité, nulle chez les animaux témoins recevant le solvant, est de 50% pour le composé de l'exemple 6, 90% pour les composés des exemples 7a et 3, et de 100% pour le composé de l'exemple 2 ; alors qu'elle est de 20% pour le bifemelane. Cette potentialisation de toxicité s'exerce à plus faible dose avec les composés de l'invention. Ainsi, elle varie de 20 à 60% à la dose de 10 mg/kg ; alors qu'aucun effet n'apparaît à cette dose pour le bifemelane.

Enfin les dérivés de l'invention ont été étudiés vis-à-vis de narcoses expérimentales. Le temps du sommeil induit par l'hexobarbital à la dose de 75mg/kg IP chez la souris est potentialisé de 62% par le bifemelane administré au préalable à la dose de 30mg/kg par voie orale, et de 86% par la viloxazine administrée au préalable à la dose de 30mg/kg par voie intrapéritonéale. Cette potentialisation est respectivement de 18 et 41% pour les composés des exemples 7a et 2 administrés préalablement à la dose de 30mg/kg per os. Malgré une activité de facilitation noradrénergique plus intense, les composés de la présente invention sont moins actifs donc moins sédatifs que les produits de référence. Cette différence essentielle apparait davantage lors du sommeil induit chez la souris par le barbital sodique (à 270mg/kg IP). En effet, les composés de l'invention diminuent significativement le temps de sommeil de 10 à 30% à la dose de 30mg/kg par voie IP ou PO, tandis que le bifemelane à la même dose augmente systématiquement de 30% la narcose barbiturique, et que la viloxazine, administrée à la dose de 30mg/kg par voie IP augmente de 6% cette narcose barbiturique. Ainsi, les composés de la présente invention sont utiles dans les cas de syndrome ischémique ou lors du vieillissement cérébral puisqu'ils s'opposent aux conséquences d'une baisse de débit sanguin ou d'oxygénation, deux des composantes pathologiques fragilisant le cerveau. Leur effet protecteur, étant dû entre autre à une amélioration de la neurotransmission noradrénergique, leur permet d'améliorer les phénomènes de baisse d'attention , d'éveil et de vigilance, ainsi que les états dépressifs et amnésiques qui accompagnent toujours l'accident vasculaire cérébral et le vieillissement.

La présente invention a également pour objet les compositions pharmaceutiques contenant comme principe actif un composé de formule générale I, ou un de ses sels physiologiquement tolérable, mélangé ou associé à un excipient pharmaceutique approprié, comme par exemple, le glucose, le lactose, l'amidon , le talc, l'éthyl cellulose, le stéarate de magnésium ou le beurre de cacao.

Les compositions pharmaceutiques ainsi obtenues se présentent généralement sous forme dosée et peuvent contenir de 1 à 100mg de principe actif. Elles peuvent revêtir, par exemple, la forme de comprimés, dragées, gélules, suppositoires, solutions injectables ou buvables et être, selon les cas, administrées par voie orale, rectale ou parentérale, à la dose de 1 à 100mg/kg, 1 à 3 fois par jour.

Les exemples suivants illustrent la présente invention ; les constantes RMN des produits obtenus étant regroupées dans le tableau 1.

**EXEMPLE 1 :**

R,S [(benzyl – 4 morpholinyl – 2) méthoxy ] – 8 tétrahydro – 1,2, 3,4 quinoléine.

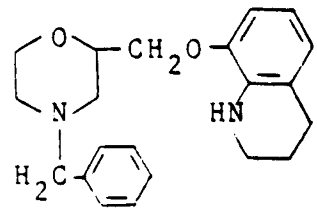

A une solution de 7,25g d'hydroxy – 8 quinoléine dans 200ml de diméthyl formamide anhydre on ajoute 2,75g d'hydrure de sodium à 50% dans l'huile, préalablement lavé au benzène, puis on chauffe le mélange pendant 2 heures à 60 ° C. On ajoute ensuite 13g de chlorométhyl – 2 benzyl – 4 morpholine

$$(Eb/_{0,05} = 120-124°C \; ; \; n_{20}^{D} = 1,5355)$$

et continue le chauffage pendant 16 heures à 110 ° C. Ensuite, on refroidit et concentre à sec. On reprend avec 50ml de chlorure de méthylène et 20ml d'eau, décante et évapore la couche organique. On obtient 6,9g de produit huileux que l'on purifie par chromatoflash sur 1250g de $SiO_2$ avec le système $CH_2CL_2$ – $CH_3OH$ (95 – 5). On obtient finalement 4,9g de [(benzyl – 4 morpholinyl – 2) méthoxy] – 8 quinoléine sous forme amorphe (dont les constantes RMN sont indiquées dans le tableau 2). On hydrogène pendant 24 heures sous une pression d'hydrogène d'environ $5.10^5$ Pa, une solution de 16,2g du produit précédemment obtenu en solution dans 486ml d'éthanol en présence de 97,2ml d'HCl N et de 1,7g de Rh/C à 5% comme catalyseur. A la fin de la réduction, on filtre le catalyseur et évapore le solvant sous pression réduite. Le résidu huileux est traité avec 50ml de $CH_2Cl_2$ et 50ml de $Na_2CO_3$ à 10%. Après décantation et évaporation de la portion organique le résidu huileux est purifié par chromatoflash sur 550g de $SiO_2$ avec le système $CH_2Cl_2 - CH_3CO - OC_2H_5$ (90 – 10). On récupère 9,9g de [(benzyl – 4 morpholinyl – 2) méthoxy] – 8 tétrahydro – 1,2,3,4 quinoléine pure, dont une partie a été transformée en dichlorhydrate par HCl,N. De la même façon, ont été préparés les produits suivants :

a) R,S [(isopropyl – 4 morpholinyl – 2) méthoxy] – 8 tétrahydro – 1,2,3,4 quinoléine et son dichlorhydrate dont le P.F. (capillaire) = 229 – 235 ° C

b) R,S [(méthyl – 4 morpholinyl – 2) méthoxy] – 8 tétrahydro – 1,2,3,4 quinoléine.

c) R,S [(éthyl – 4 morpholinyl – 2) méthoxy] – 8 tétrahydro – 1,2,3,4 quinoléine.

d) R,S [(propyl – 4 morpholinyl – 2) méthoxy] – 8 tétrahydro – 1,2,3,4, quinoléine.

e) R,S [(cyclopentyl – 4 morpholinyl – 2) méthoxy] – 8 tétrahydro – 1,2,3,4 quinoléine, P.F. (capillaire) du dichlorohydro correspondant = 198 – 205 ° C.

f) R,S [(p.chlorobenzyl – 4 morphonilyl – 2) méthoxy] – 8 tétrahydro – 1,2,3,4 quinoléine.

**EXEMPLE 2 :**

R,S [(morpholinyl – 2) méthoxy] – 8 tétrahydro – 1,2,3,4 quinoléine

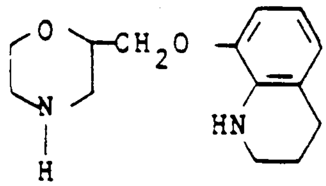

Une solution de 5g de R,S [(benzyl – 4 morpholinyl – 2) méthoxy] – 8 tétrahydro – 1,2,3,4 quinoléine (préparée selon l'exemple 1) dans 100ml d'éthanol est soumise à hydrogénolyse sous une pression d'hydrogène d'environ $4.10^5$ Pa, en présence de 0,4g de $Pd(OH)_2$ à 20% sur C, pendant 20 heures à 50 ° C. Au bout de ce temps, on filtre le catalyseur et évapore le solvant sous pression réduite. Le résidu huileux est chromatographié sur 150g de $SiO_2$ avec le système $CH_2Cl_2 - CH_3OH$ (70 – 30). On recueille finalement sous forme amorphe 3,9g de [(morpholinyl – 2) méthoxy] – 8 tétrahydro – 1,2,3,4 quinoléine pure, que l'on transforme en dichlorhydrate par HCl,N ; P.F. (capillaire) : 228 – 231 ° C.

De la même façon ont été préparés les produits suivants :

a) $S^{(+)}$ [(morpholinyl – 2) méthoxy] – 8 tétrahydro – 1,2,3,4 quinoléine, et son dichlorhydrate ; en opérant comme suit :

α) On chauffe pendant 20 heures à 110 ° C, une solution de 57g. de $S^{(+)}$ tosyloxyméthyl – 2 benzyl – 4 morpholine et de 32g. de sel de potassium d'hydroxy – 8 quinoléine (préparé extemporanément à partir d'une solution équimoléculaire d'hydroxy – 8 quinoléine et de lessive de potasse, dans l'éthanol) dans 900ml de diméthylformamide. A la fin de la réaction, on évapore le solvant sous pression réduite et reprend le résidu dans 300ml de $CH_2Cl_2$. La solution organique est lavée avec de la soude 0,5N, lavée à l'eau et séchée sur $Na_2SO_4$. Après évaporation du solvant, le résidu huileux est purifié par chromatoflash sur 1200g de silice avec l'acétate d'éthyle comme éluant. On obtient après concentration des fractions 50,3 g de $S^{(+)}$ [(benzyl – 4 morpholinyl – 2) méthoxy] – 8 quinoléine,

$$\alpha_D^{25,5} = + 6^{\circ},5 \ (C=2\ MeOH).$$

En opérant de la même façon, à partir de $R^{(-)}$ tosyloxyméthyl – 2 benzyl – 4 morpholine, on obtient la $R^{(-)}$ [(benzyl – 4 morpholinyl – 2) méthoxy] – 8 quinoléine,

$$\alpha_D^{25,5} = - 6^{\circ},4 \ (C=2\ MeOH)$$

β) On dissout 48,2g de $S^{(+)}$ [(benzyl – 4 morpholinyl – 2) méthoxy] – 8 quinoléine précédemment obtenue dans 900ml de méthanol, on ajoute 288ml d'HCl,N et on hydrogène le cycle quinoléinique sous $6.10^5$ Pa d'hydrogène en présence de 4,5g. de Rhodium/C à 5%. Au bout de 2 heures la quantité théorique d'hydrogène étant absorbée, on filtre le catalyseur, ajoute 4,5g de Palladium/C à 10%, et hydrogène à 60 ° C sous $6.10^5$ Pa d'hydrogène. La quantité théorique d'hydrogène étant obsorbée au bout de 6 heures, on filtre alors et évapore le solvant, reprend le résidu huileux par 300ml de $CH_2$ $Cl_2$ et lave avec une solution à 10% de $Na_2CO_3$. On décante et sèche la phase organique sur $Na_2SO_4$, puis évapore et purifie le résidu par chromatoflash sur 1260g de silice (éluant: $CH_2Cl_2$ – méthanol, 80 – 20). Les fractions collectées sont concentrées sous pression réduite. On obtient 31g de base résineuse qu'on dissout dans 200ml d'éthanol. Après addition d'HCl sec, à pH acide, on ajoute 600ml d'éther anhydre. On essore 38g de produit amorphe que l'on cristallise dans 77,6ml de propanol additionné de 3,88ml d'eau. On obtient finalement 32,5g de dichlorhydrate de $S^{(+)}$ [(morpholinyl – 2 méthoxy] – 8 tétrahydro – 1,2,3,4 quinoléine, fondant (capillaire) à 174 – 178 ° C ;

$$\alpha_D^{25,5} = +8°,4 \text{ (C=2 pyridine).}$$

b) $R^{(-)}$[(morpholinyl-2) méthoxy]-8 tétrahydro-1,2,3,4 quinoléine, et son dichlorhydrate dont les constantes physiques sont : PF (capillaire) = 173-175 °C ;

$$\alpha_D^{22,5} = -8,85° \text{ (C=2 pyridine),}$$

préparé selon le protocole décrit dans l'exemple 2a, à partir de la $R^{(-)}$ [(benzyl-4 morpholinyl-2) méthoxy]-8 quinoléine,

$$\alpha_D^{25,5} = -6,4° \text{(C=2 méthanol),}$$

elle-même préparée à partir de la $R^{(-)}$ tosyloxyméthyl-2 benzyl-4 morpholine.

Les $S^{(+)}$ et $R^{(-)}$ tosyloxyméthyl-2 benzyl-4 morpholine, matières premières des exemples 2a et 2b ont été préparées à partir de la R,S tosyloxyméthyl-2 benzyl-4 morpholine, matière première de l'exemple 2, elle-même préparée de la manière suivante :

On dissout 62,15g de R,S hydroxy méthyl-2 benzyl-4 morpholine dans 1 litre de pyridine. On refroidit à +5 °C et ajoute 57,2g de chlorure de tosyle sans dépasser +10 °C. On abandonne la solution au réfrigérateur pendant 20 heures, puis évapore la pyridine sous pression réduite à 30 °C.

On reprend le résidu avec $CH_2Cl_2$, lave avec une solution à 10% de $Na_2CO_3$, sèche sur $Na_2SO_4$ et évapore le solvant sous pression réduite. On reprend le résidu avec de l'heptane. Le produit cristallise. On essore et recueille 90,7g de cristaux blancs fondant (Kofler) à 74 °C. La séparation des énantiomères a été effectuée, selon la méthode de R.HOWE et coll. J. Med. Chem. (1976), 19, 1674-76, à partir d'acide tosyl S glutamique, P.F. (Kofler) : 125 °C.

les constantes physiques des énantiomères obtenus sont :

Isomère $S^{(+)}$ : $P.F._{(K)}$ :

$$70°C, \ \alpha_D^{21} = -19,8° \text{ (C=2 méthanol)}$$

Isomère $R^{(-)}$ : $P.F._{(K)}$ :

$$71°C, \ \alpha_D^{21} = +21° \text{ (C=2 méthanol)}$$

**EXEMPLE 3 :**

R,S méthyl – 1[(benzyl – 4 morpholinyl – 2) méthoxy] – 8 tétrahydro – 1,2,3,4 quinoléine.

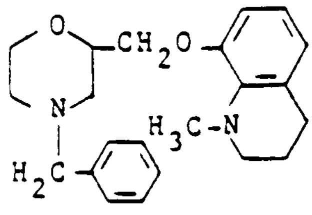

A une solution de 9,6g de R,S [(benzyl – 4 morpholinyl – 2) méthoxy] – 8 tétrahydro – 1,2,3,4 quinoléine (préparée selon l'exemple 1) dans 100ml d'acétone, on ajoute 4,5g d'iodure de méthyle et 4,3g de $K_2CO_3$ et chauffe le mélange pendant 3 heures à reflux. Ensuite, on filtre le sel et évapore le solvant sous pression réduite. Le résidu est repris par 50ml de $CH_2Cl_2$ et 50ml d'eau. Après décantation, la portion organique est évaporée et le résidu huileux est chromatographié sur 850g de $SiO_2$ avec le système $CH_2Cl_2$ – $CH_3OH$ (97 – 3). On isole 4g de R,S méthyl – 1 [(benzyl – 4 morpholinyl – 2) méthoxy] – 8 tétrahydro – 1,2,3,4, quinoléine, sous forme amorphe ; qui a été transformée en dichlorhydrate au sein d'HCl,N.

De la même façon ont été préparés les produits suivants :

a) R,S isopropyl – 1 [(isopropyl – 4 morpholinyl – 2) méthoxy] – 8 tétrahydro – 1,2,3,4 quinoléine, et son dichlorhydrate

b) R,S isopropyl – 1 [(benzyl – 4 morpholinyl – 2) méthoxy] – 8 tétrahydro – 1,2,3,4 quinoléine.

c) R,S isobutyl – 1 [(benzyl – 4 morpholinyl – 2) méthoxy] – 8 tétrahydro – 1,2,3,4 quinoléine.

d) R,S méthyl – 1 [(isopropyl – 4 morpholinyl – 2) méthoxy] – 8 tétrahydro – 1,2,3,4 quinoléine, et son dichlorhydrate dont le P.F. (capillaire) = 228 – 231 ° C.

**EXEMPLE 4 :**

R,S isopropyl – 1 [(isopropyl – 4 morpholinyl – 2) méthoxy] – 8 tétrahydro – 1,2,3,4 quinoléine.

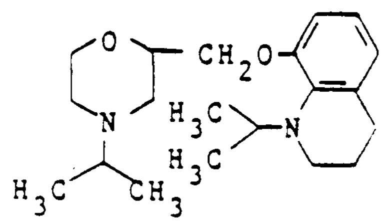

On chauffe pendant 20 heures à reflux une solution de 8,1g de R,S [(morpholinyl – 2 – méthoxy] – 8 tétrahydro – 1,2,3,4 quinoléine (préparée selon l'exemple 2) dans 170ml d'éthanol avec 19,5g d'iodure d'isoproyle et 10ml de triéthylamine.

Au bout de ce temps, on chasse le solvant sous pression réduite et reprend le résidu par 50ml de $CH_2Cl_2$ et 50ml d'eau, puis on décante et évapore le solvant. Le résidu huileux est chromatographié sur 600g de $SiO_2$ avec le système $CH_2Cl_2$ – $CH_3OH$ (96 – 4). On isole 7,5g d'isopropyl – 1 [(isopropyl – 4 morpholinyl – 2) méthoxy] – 8 tétrahydro – 1,2,3,4 quinoléine, sous forme amorphe qui sont transformés en dichlorhydrate (également amorphe) au sein d'HCl,N.

De la même façon ont été préparés les produits suivants :

a) R,S méthyl – 1 [(méthyl – 4 morpholinyl – 2) méthoxy] – 8 tétrahydro – 1,2,3,4 quinoléine.

b) R,S éthyl – 1 [(éthyl – 4 morpholinyl – 2) méthoxy] – 8 tétrahydro – 1,2,3,4 quinoléine.

c) R,S isobutyl – 1[(isobutyl – 4 morpholinyl – 2) méthoxy] – 8 tétrahydro – 1,2,3,4 quinoléine.

d) R,S benzyl – 1 [(benzyl – 4 morpholinyl – 2 méthoxy] – 8 tétrahydro – 1,2,3,4 quinoléine.

e) R,S allyl – 1 [(allyl – 4 morpholinyl – 2) méthoxy] – 8 tétrahydro – 1,2,3,4 quinoléine.

**EXEMPLE 5 :**

R,S acétyl – 1 [(benzyl – 4 morpholinyl – 2) méthoxy] – 8 tétrahydro – 1,2,3,4 quinoléine.

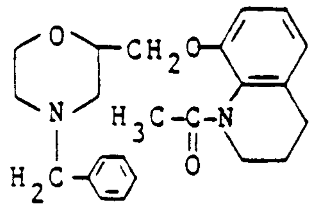

A une solution de 12,5g de R,S [(benzyl – 4 morpholinyl – 2) méthoxy] – 8 tétrahydro – 1,2,3,4 quinoléine (préparée selon l'exemple 1) dans 100ml de tétrahydrofuranne anhydre contenant 5,5ml de triéthylamine, on ajoute, à la température de 5 ° C, 2,86g de chlorure d'acétyle. on agite pendant 1 heure à cette température, puis on chauffe pendant 1 heure et demie a 45 ° C. On évapore ensuite le solvant et reprend le résidu par 50ml de $CH_2Cl_2$ et 50ml de carbonate de sodium à 10%. On décante et évapore la portion organique. Le résidu pesant 14,3g est purifié par chromatoflash sur 2,50g de $SiO_2$ avec le système $CH_2Cl_2$ – $CH_3OH$ (95 – 5). On isole finalement 13,3g de R,S acétyl – 1 [(benzyl – 4 morpholinyl – 2) méthoxy] – 8 tétrahydro – 1,2,3,4 quinoléine, sous forme de résine, qui sont transformés en chlorhydrate amorphe. De la même façon, ont été préparés les produits suivants :

a) R,S acétyl – 1 [(acétyl – 4 morpholinyl – 2) méthoxy] – 8 tétrahydro – 1,2,3,4 – quinoléine.

b) R,S acétyl – 1 [(isopropyl – 4 morpholinyl – 2) méthoxy] – 8 tétrahydro – 1,2,3,4 quinoléine, et son chlorhydrate.

c) R,S [(acétyl – 4 morpholinyl – 2) méthoxy] – 8 tétrahydro – 1,2,3,4 quinoléine, et son chlorhydrate.

**EXEMPLE 6:**

R,S acétyl – 1 [(morpholinyl – 2) méthoxy] – 8 tétrahydro – 1,2,3,4 quinoléine.

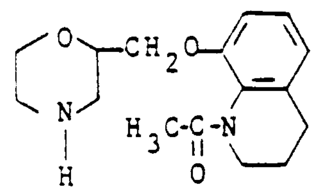

On soumet à hydrogénation à température ambiante, en présence de 0,86g de $Pd(OH)_2$ 20%/C, une solution de 8,6g de R,S – acétyl – 1 [(benzyl – 4 morpholinyl – 2) méthoxy] – 8 tétrahydro – 1,2,3,4 quinoléine, (préparée selon l'exemple 5) dans 170ml d'éthanol, sous une pression d'hydrogène d'environ $6.10^5$ Pa. Au bout de 20 heures, la quantité théorique d'hydrogène est absorbée. On filtre le catalyseur et concentre la solution sous pression réduite. On chromatographie le résidu sur 235g de $SiO_2$ avec le système $CH_2Cl_2$ – $CH_3OH$ (85 – 15). On recueille 5,7g de R,S – acétyl – 1 [(morpholinyl – 2) méthoxy] – 9 tétrahydro – 1,2,3,4 quinoléine sous forme de résine que l'on transforme en chlorhydrate amorphe.

**EXEMPLE 7 :**

R,S [(allyl-4 morpholinyl-2) méthoxy]-8 tétrahydro-1,2,3,4 quinoléine.

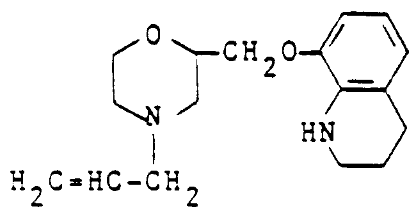

En opérant comme dans l'exemple 4 à partir d'acétyl-1 [(morpholinyl-2) méthoxy]-8 tétrahydro-1,2,3,4 quinoléine (selon l'exemple 6) et du bromure d'allyle on obtient l'acétyl-1 [(allyl-4 morpholinyl-2) méthoxy]-8 tétrahydro-1,2,3,4 quinoléine, laquelle hydrolysée par Na OH en solution éthanolique donne le R,S[(allyl-4 morpholinyl-2) méthoxy]-8 tétrahydro-1,2,3,4 quinoléine, P.F. (Kofler) du dichlorhydre correspondant = 188°C.

De la même façon ont été préparés les produits suivants :

a) R,S [(isopropyl-4 morpholinyl-2) méthoxy]-8 tétrahydro-1,2,3,4 quinoléine, et son dichlorhydrate.

b) R,S [(isobutyl-4 morpholinyl-2) méthoxy]-8 tétrahydro-1,2,3,4 quinoléine.

c) R,S [(p.chlorobenzyl-4 morpholinyl-2) méthoxy]-8 tétrahydro-1,2,3,4 quinoléine.

d) R,S méthyl-1 (mopholinyl-2 méthoxy)-8 tétrahydro-1,2,3,4 quinoléine, et son dichlorhydrate dont le P.F. (capillaire) = 193-198°C.

e) R,S [(pipéronyl-4 morpholinyl-2) méthoxy]-8 tétrahydro-1,2,3,4 quinoléine, et son dichlorhydrate (amorphe)

f) $S^{(+)}$ [(isopropyl-4 morpholinyl-2) méthoxy]-8 tétrahydro-1,2,3,4 quinoléine et son dichlorhydrate dont le P.F. (capillaire) = 140-145°C et

$$\alpha_D^{22} = +30,7° \text{ (C=2 pyridine)}$$

préparé selon la méthode de l'exemple 7 à partir de $S^{(+)}$ [(isopropyl-4 morpholinyl-2) méthoxy]-8 acétyl-1 tétrahydro-1,2,3,4 quinoléine, $^{22}_{D}$ = +13,9° (C=2 pyridine), elle-même préparée à partir de l'iodure d'isopropyle et de $S^{(+)}$ (morpholinyl-2 méthoxy)-8 acétyl-1 tétrahydro-1,2,3,4 quinoléine, $\alpha_D^{22}$ = +13,5° (C=5 pyridine) elle-même préparée, selon la méthode de l'exemple 6, par débenzylation de $S^{(+)}$ [(benzyl-4 morpholinyl-2) méthoxy]-8 acétyl-1 tétrahydro-1,2,3,4 quinoléine, $\alpha_D^{22}$ = +24,7° (C=2 pyridine) elle-même préparée selon la méthode de l'exemple 5, à partir de chlorure d'acétyle et de $S^{(+)}$ [(benzyl-4 morpholinyl-2) méthoxy]-8 tétrahydro-1,2,3,4 quinoléine, $\alpha_D^{22}$ = +21° (C=2 pyridine), elle-même préparée selon la méthode de l'exemple 1.

g) $R^{(-)}$ [(isopropyl-4 morpholinyl-2) méthoxy]-8 tétrahydro-1,2,3,4 quinoléine, et son dichlorhydrate dont le P.F. (capillaire) = 135-140°C et $\alpha_D^{22}$ = -29,5° (C=2 pyridine) préparé selon la méthode de l'exemple 7 à partir de $R^{(-)}$ [(isopropyl-4 morpholinyl-2) méthoxy]-8 acétyl-1 tétrahydro-1,2,3,4 quinoléine, $\alpha_D^{22}$ = -14°C (C=2 pyridine), elle-même préparée à partir de l'iodure d'isopropyle et de $R^{(-)}$ (morpholinyl-2 méthoxy)-8 acétyl-1 tétrahydro-1,2,3,4 quinoléine, $\alpha_D^{22}$ = -14,9° (C=2 pyridine), elle-même préparée selon la méthode de l'exemple 6, par débenzylation de $R^{(-)}$ [(benzyl-4 morpholinyl-2) méthoxy]-8 acétyl-1 tétrahydro-1,2,3,4 quinoléine, $\alpha_D^{22}$ = -24,7° (C=2 pyridine), elle-même préparée selon la méthode de l'exemple 5, à partir de chlorure d'acétyle et de $R^{(-)}$ [-(benzyl-4 morpholinyl-2) méthoxy]-8 tétrahydro-1,2,3,4 quinoléine, $\alpha_D^{22}$ = -21° (C=2 pyridine), elle-même préparée selon la méthode de l'exemple 1.

Les constantes RMN des produits précédemment exemplifiés sont regroupés dans le tableau ci-après.

TABLEAU 1

RMN des produits objet des exemples précédents

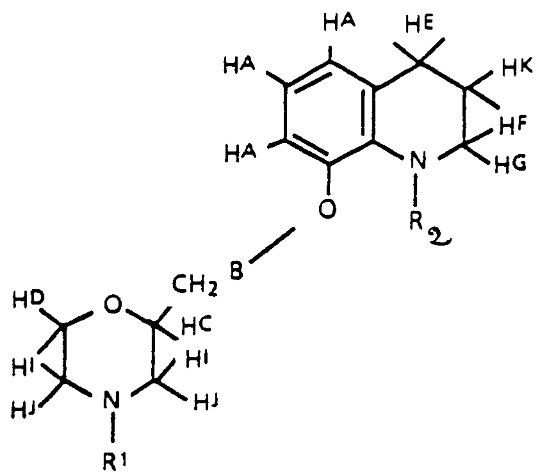

$R_2=H;R_1=CH_2C_6H_5$ (Exemple 1)

($CDCl_3$) δ = 7,3 (s, 5H, $CH_2C_6\underline{H_5}$) ; 6,3 à 6,8 (m, 3H, $H^A$) ; 3,6 à 4,3 (m, 5H, $H^B$, $H^C$, $H^D$) 3,5 (s, 2H, $\underline{CH_2}$, $C_6H_5$) 3,25 (t, 2H, $H^F$, $H^G$); 1,7 à 3,1 (m, 8H, $H^E$, $H^I$, $H^J$, $H^K$); 1 proton N$\underline{H}$ échangeable par $D_2O$ vers 3,4 ppm.

$R_2=R_1=H$ (Exemple 2)

($CDCl_3$) δ = 6,4 à 6,8 (m, 3H, $H^A$) ; 3,6 à 4,2 (m, 5H, $H^B$, $H^C$, $H^D$) ; 2,4 à 3,6 (m, 10H dont deux échangeables par $D_2O$ : $R_2$ et $R_1$ ; $H^E$, $H^F$, $H^G$, $H^I$, $H^J$); 1,9 (m, 2H, $H^K$)

$R_2=CH_3;R_1=CH_2C_6H_5$ (Exemple 3)

($CDCl_3$) δ = 7,3 (s, 5H, $CH_2C_6\underline{H_5}$) 6,5 à 7,1 (m, 3H, $H^A$) 3,5 à 4,4 (m, 5H, $H^B$, $H^C$, $H^D$) 3,5 (s, 2H, $C\underline{H_2}C_6H_5$) 2,5 à 3,4 (m, 6H, $H^E$, $H^F$, $H^G$, $H^I$) 2,7 (s, 3H, N-$C\underline{H_3}$) 1,4 à 2,5 (m, 4H, $H^K$, $H^J$)

$R_2=R_1=(CH(CH_3)_2$ (Exemple 3a)

($CDCl_3$) δ = 6,6 à 7,1 (m, 3H, $H^A$) 3,6 à 4,5 (m, 5H, $H^B$, $H^C$, $H^D$) ; 2,1 à 3,6 (m, 10H, $H^E$, $H^F$, $H^G$, $H^I$, $H^J$ ; 2$C\underline{H}$ $(CH_3)_2$) 1,9 (m, 2H, $H^K$) 1,15 - 1,05 (deux doublets, 12H, 2CH($\underline{CH_3}$)$_2$)

$R_2=COCH_3;R_1=CH_2C_6H_5$ (Exemple 5)

($CDCl_3$) δ = 7,4 (s, 5H, $CH_2C_6\underline{H_5}$) 6,6 à 7,3 (m, 3H, $H^A$) 4,6 (m, 1H, $H^G$) 3,6 à 4,2 (m, 5H, $H^B$, $H^C$, $H^D$) 3,6 (s, 2H, $\underline{CH_2}C_6H_5$) 1,4 à 3,2 (m, 9H, $H^E$, $H^F$, $H^I$, $H^J$, $H^K$) 2,0 (s, 3H, $CO\underline{CH_3}$)

$R_1$=$R_2$=$COCH_3$ (Exemple 5a)

($CDCl_3$) S = 6,6 à 7,6 (m, 3H, HA) ; 4,5 (m, 3H, HG, HJ) 3,35 à 4,2 (m, H, HB, HC, HD) 2,2 à 3,35 (m, 5H, HE, HF, HI) 2,0 et 2,1 (deux singulets 6H ; 2COC<u>H</u>3) 1,8 (m, 2H, HK)

$R_2$=$COCH_3$;$R_1$=$CH(CH_3)_2$ (Exemple 5b)

($CDCl_3$) S = 6,3 à 7,4 (m, 3H, HA) 4,5 (m, 1H, HG) 3,3 à 4,1 (m, 5H, HB, HC, HD) 2,0 à 3,3 (m, 8H, HE, HF, HI, HJ ; <u>CH</u>($CH_3$)$_2$) 2,0 (s, 3H, CO<u>CH</u>$_3$) 1,9 (m, 2H, HK) ; 1,1 (d, 6H, CH(<u>CH</u>$_3$)$_2$)

$R_2$=H;$R_1$=$COCH_3$ (Exemple 5c)

($CDCl_3$) S = 6,4 à 6,9 (m, 3H, HA) ; 4,5 (m, 2H, HJ) 2,9 à 4,1 (m, 9H, HB, HC, HD, HI, HF, HG) 2,8 (t, 2H, HE) 2,1 (s, 3H, CO<u>CH</u>$_3$) 1,9 (m, 2H, HK) 1 proton NH, non mis en évidence

$R_2$=$COCH_3$;$R_1$=H (Exemple 6)

($CDCl_3$) S = 6,3 à 7,5 (m, 3H, HA) ; 4,5 (m, 1H, HG) ; 3,5 à 4,1 (m, 5H, HB, HC, HD) ; 2,2 à 3,5(m, 8H dont un échangeable par $D_2O$ : $R_1$ ; HE, HF, HG, HI, HJ); 2,0 (s, 3H, CO<u>CH</u>$_3$) 1,9 (m, 2H, HK)

$R_2$=H;$R_1$=$CH(CH_3)_2$ (Exemple 7a)

($CDCl_3$) S = 6,5 à 7,1 (m, 3H, HA) ; 4,6 (m, 1H échangeable par $D_2O$ $R_1$= <u>H</u>) ; 3,6 à 4,4 (m, 5H, HB, HC, HD) ; 3,4 (t, 2H, HF, HG) 1,6 à 3,1 (m, 9H, HI, HJ, HE, HK ; C<u>H</u>($CH_3$)$_2$) 1,1 (d, 6H, CH(<u>CH</u>$_3$)$_2$)

TABLEAU 2

RMN de la [(benzyl-4 morpholinyl-2) méthoxy]-8 quinoléine. (Produit intermédiaire dans l'exemple 1)

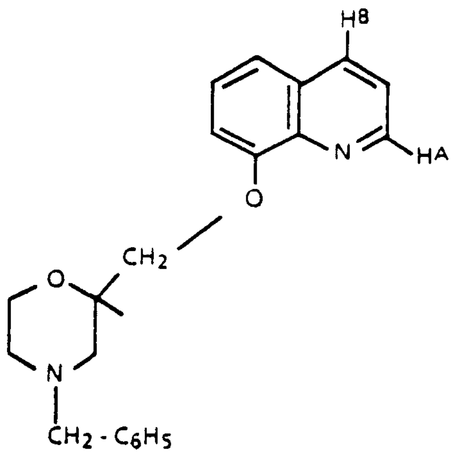

($CDCl_3$) δ = 8,9 (d, dédoublé 1H, $H^A$) 8,1 (d, dédoublé, 1H, $H^B$) 7,3 (s, 5H, $CH_2C_6\underline{H_5}$) 6,9 à 7,3 (m 4H aromatiques) 3,6 à 4,5 (m, 5H, $2C\underline{H_2}$-O et $C\underline{H}$ -O) 3,5 (s, 2H, $C\underline{H_2}C_6H_5$) 1,9 à 3,2 (m, 4H 2N - $C\underline{H_2}$)

## Revendications

**1.** Les dérivés de la morpholine de formule générale I :

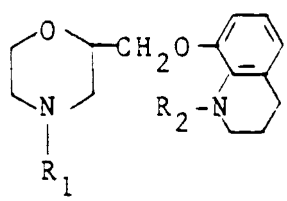

dans laquelle :
$R_1$ représente :
un atome d'hydrogène ;
un radical alkyle, contenant de 1 à 6 atomes de carbone, en chaîne droite ou ramifiée comportant éventuellement une double liaison ;
un radical aralkyle de formule générale :

$Ar-(CH_2)_m-$

dans laquelle :
Ar représente un radical aryle éventuellement substitué par un ou plusieurs atomes d'halogène, radicaux alkyle ou alkoxy contenant chacun de 1 à 5 atomes de carbone, ou radicaux de formule $-O-(CH_2)_n-O-$ dans laquelle n prend les valeurs 1 ou 2 ; et
m représente un nombre entier de 1 à 3 ;

un radical cycloalkyle contenant 5 ou 6 atomes de carbone ; ou
un radical acyle de formule : R' – CO – dans laquelle R' représente un radical alkyle renfermant 1 ou 2 atomes de carbone ; et
$R_2$ représente :
un atome d'hydrogène ;
un radical alkyle, contenant de 1 à 6 atomes de carbone, en chaîne droite ou ramifiée, comportant éventuellement une double liaison ;
un radical aralkyle de formule générale : $Ar-(CH_2)_m-$ dans laquelle Ar et m sont tels que précédemment définis ; ou
un radical acyle de formule R' – CO – dans laquelle R' prend la signification énoncée précédemment ; et
leurs énantiomères.

2. Les sels physiologiquement tolérables des composés de la revendication 1 avec des acides appropriés.

3. La R,S [(benzyl – 4 morpholinyl – 2) méthoxy] – 8 tétrahydro – 1,2,3,4 quinoléine.

4. La R,S [(cyclopentyl – 4 morpholinyl – 2) méthoxy] – 8 tétrahydro – 1,2,3,4 quinoléine.

5. La R,S [(p. chlorobenzyl – 4 morpholinyl – 2) méthoxy] – 8 tétrahydro – 1,2,3,4 quinoléine.

6. La R,S [(morpholinyl – 2) méthoxy] – 8 tétrahydro – 1,2,3,4 quinoléine, son dichlorhydrate et ses énantiomères.

7. La R,S méthyl – 1 [(benzyl – 4 morpholinyl – 2) méthoxy] – 8 tétrahydro – 1,2,3,4 quinoléine.

8. La R,S isopropyl – 1 [(isopropyl – 4 morpholinyl – 2) méthoxy] – 8 tétrahydro – 1,2,3,4 quinoléine.

9. La R,S acétyl – 1 [(isopropyl – 4 morpholinyl – 2) méthoxy] – 8 tétrahydro – 1,2,3,4 quinoléine.

10. La R,S acétyl – 1 [(morpholinyl – 2) méthoxy] – 8 tétrahydro – 1,2,3,4 quinoléine.

11. La R,S [(allyl – 4 morpholinyl – 2) méthoxy] – 8 tétrahydro – 1,2,3,4 quinoléine.

12. La R,S [(isopropyl – 4 morpholinyl – 2) méthoxy] – 8 tétrahydro – 1,2,3,4 quinoléine, son dichlorhydrate et ses énantiomères.

13. Le procédé de préparation des composés de la revendication 1, caractérisé en ce que :
l'on fait réagir une morpholine substituée de formule générale II.

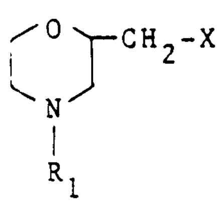

(II)

dans laquelle :
$R_1$ prend la signification définie dans la revendication 1, et
X représente un atome d'halogène ou un radical tosyloxy,
avec l'hydroxyquinoléine de formule III

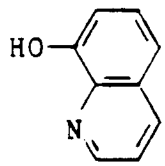

(III)

préalablement transformée en sel alcalin,
pour obtenir un composé de formule générale IV

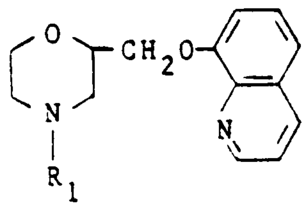

(IV)

dans laquelle $R_1$ a la signification énoncée dans la revendication 1 ;
que l'on hydrogène ensuite pour obtenir un dérivé de formule Ia

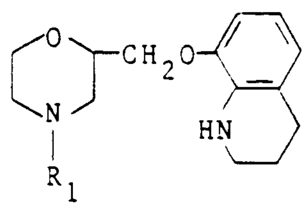

(Ia)

dans laquelle $R_1$ est tel que défini dans la revendication 1,
lequel est alors condensé avec un dérivé de formule générale V

$R'_2-X'$ (V)

dans laquelle :
$R'_2$ représente
un radical alkyle contenant de 1 à 6 atomes de carbone, en chaîne droite ou ramifiée, comportant éventuellement une double liaison,
un radical aralkyle de formule générale : $Ar-(CH_2)_m-$, dans lequel Ar et m sont tels que définis dans la revendication 1, et
simultanément X' représente un atome de brome un d'iode ou un radical tosyloxy ; ou
$R'_2$ représente un radical acyle de formule $R'-CO-$ dans laquelle R' a la signification énoncée dans la revendication 1, et
simultanément X' représente un atome de chlore ;
pour obtenir un dérivé de formule générale Ib :

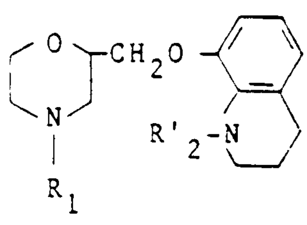

(Ib)

dans laquelle $R_1$ et $R'_2$ ont les significations énoncées précédemment.

**14.** Le procédé de préparation selon la revendication 13 caractérisé en ce que la réaction des dérivés II et III est effectuée dans un solvant choisi parmi les amides tertiaires, à une température comprise entre 90 et 110 ° C en présence d'un accepteur de l'hydracide formé au cours de la réaction.

**15.** Un procédé selon la revendication 14 caractérisé en ce que l'accepteur est l'hydrure de sodium.

**16.** Un procédé selon la revendication 13 caractérisé en ce que l'hydrogénation du composé IV s'effectue à basse pression en présence d'un catalyseur appartenant aux métaux du VIIIème groups, dans un solvant tel que les alcools à bas poids moléculaire, à une température comprise entre 20 et 60 ° C.

**17.** Le procédé selon la revendication 16 caractérisé en ce que l'hydrogénation est réalisée, sous une pression d'hydrogène de $1.10^5$ à $6.10^5$ Pa, en présence de Rh/C ou $Pd(OH)_2/C$ comme catalyseur.

**18.** Le procédé selon la revendication 13 caractérisé en ce que la condensation des composés (Ia) et V est réalisée dans un solvant choisi parmi les hydrocarbures aromatiques ou les solvants polaires, à une température comprise entre 20 et 80 ° C en présence d'un accepteur de l'hydracide formé.

**19.** Le procédé selon la revendication 18 caractérisé en ce que l'accepteur est une amine tertiaire.

**20.** Le procédé de préparation des composés de la revendication 1 répondant à la formule générale I' :

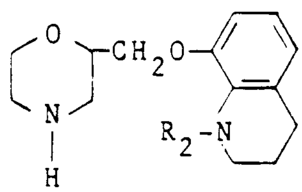

(I')

dans laquelle $R_2$ est tel que défini dans la revendication 1 caractérisé en ce que l'on condense une morpholine de formule générale II' :

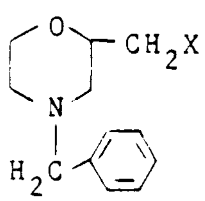

(II')

dans laquelle X est tel que défini dans la revendication 13, avec l'hydroxyquinoléine de formule III :

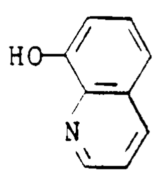

(III)

pour obtenir un dérivé de formule IV' :

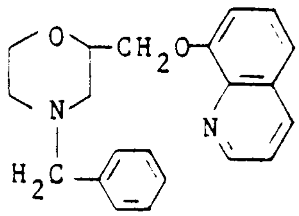

(IV')

lequel est soumis à une hydrogénation catalytique sous basse pression pour obtenir un dérivé de formule IV" :

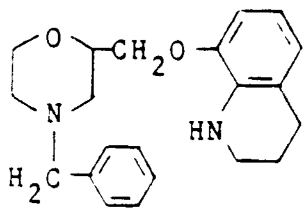

(IV")

lequel est :
soit débenzylé directement par hydrogénolyse, pour donner le dérivé de formule I'a

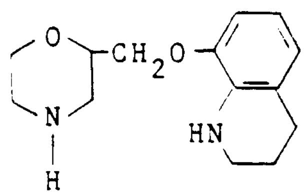

(I'a)

soit d'abord alkylé ou acylé au moyen de $R'_2X'$ tel que défini dans la revendication 13 puis débenzylé par hydrogénation catalytique sous basse pression pour obtenir le dérivé de formule générale I'b

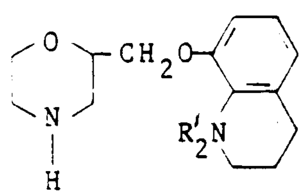

(I'b)

dans laquelle $R'_2$ a la signification définie dans la revendication 13.

**21.** Les compositions pharmaceutiques contenant comme principe actif un composé selon les revendications 1 à 12, avec des excipients pharmaceutiques appropriés.

**22.** Les compositions pharmaceutiques selon la revendication 21, présentées sous une forme convenant notamment pour le traitement des syndromes ischémiques et du vieillissement cérébral.

## Claims

1. Morpholine compounds of the general formula I:

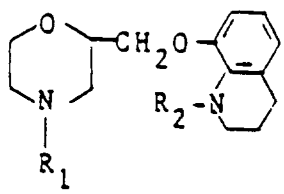

in which:
$R_1$ represents:
a hydrogen atom;
a straight-chain or branched alkyl radical containing from 1 to 6 carbon atoms and optionally having a double bond;
an aralkyl radical of the general formula:

$Ar-(CH_2)_m-$

in which:
Ar represents an aryl radical optionally substituted by one or more halogen atoms, alkyl or alkoxy radicals each containing from 1 to 5 carbon atoms, or radicals of the formula $-O-(CH_2)_n-O-$ in which n has the value 1 or 2; and
m represents an integer from 1 to 3;
a cycloalkyl radical containing 5 or 6 carbon atoms; or
an acyl radical of the formula: R'-CO- in which R' represents an alkyl radical containing 1 or 2 carbon atoms; and
$R_2$ represents:
a hydrogen atom;
a straight-chain or branched alkyl radical containing from 1 to 6 carbon atoms and optionally having a double bond;
an aralkyl radical of the general formula: $Ar-(CH_2)_m-$ in which Ar and m are as defined above; or
an acyl radical of the formula R'-CO- in which R' is as defined above;
and their enantiomers.

2. The physiologically tolerable salts of the compounds of claim 1 with suitable acids.

3. R,S-8-[(4-benzyl-2-morpholinyl)-methoxy]-1,2,3,4-tetrahydroquinoline.

4. R,S-8-[(4-cyclopentyl-2-morpholinyl)-methoxy]-1,2,3,4-tetrahydroquinoline.

5. R,S-8-[(4-p-chlorobenzyl-2-morpholinyl)-methoxy]-1,2,3,4-tetrahydroquinoline.

6. R,S-8-[(2-morpholinyl)-methoxy]-1,2,3,4-tetrahydroquinoline, its dihydrochloride and its enantiomers.

7. R,S-1-methyl-8-[(4-benzyl-2-morpholinyl)-methoxy]-1,2,3,4-tetrahydroquinoline.

8. R,S-1-isopropyl-8-[(4-isopropyl-2-morpholinyl)-methoxy]-1,2,3,4-tetrahydroquinoline.

9. R,S-1-acetyl-8-[(4-isopropyl-2-morpholinyl)-methoxy]-1,2,3,4-tetrahydroquinoline.

10. R,S-1-acetyl-8-[(2-morpholinyl)-methoxy]-1,2,3,4-tetrahydroquinoline.

11. R,S-8-[(4-allyl-2-morpholinyl)-methoxy]-1,2,3,4-tetrahydroquinoline.

**12.** R,S-8-[(4-isopropyl-2-morpholinyl)-methoxy]-1,2,3,4-tetrahydroquinoline, its dihydrochloride and its enantiomers.

**13.** Process for the preparation of the compounds of claim 1, characterised in that:
a substituted morpholine of the general formula II:

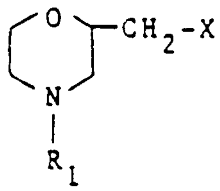

(II)

in which:
$R_1$ is as defined in claim 1, and
X represents a halogen atom or a tosyloxy radical,
is reacted with a hydroxyquinoline of the formula III:

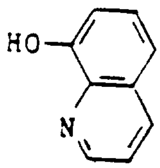

(III),

which has previously been converted into an alkali metal salt,
to obtain a compound of the general formula IV:

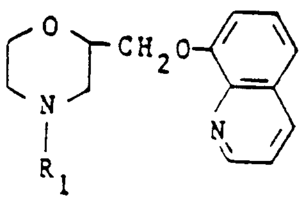

(IV)

in which $R_1$ is as defined in claim 1;
which is then hydrogenated to obtain a compound of the formula Ia:

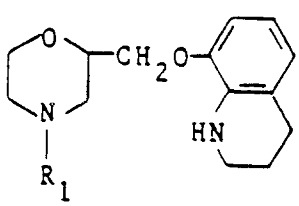

(Ia)

in which $R_1$ is as defined in claim 1,
which is then condensed with a compound of the general formula V:

$R'_2-X'$ (V)

in which:
$R'_2$ represents
a straight-chain or branched alkyl radical containing from 1 to 6 carbon atoms and optionally having a double bond,
an aralkyl radical of the general formula: $Ar-(CH_2)_m-$, in which Ar and m are as defined in claim 1,

and,
at the same time, X' represents a bromine or iodine atom or a tosyloxy radical; or
$R'_2$ represents an acyl radical of the formula R' − CO − in which R' is as defined in claim 1, and, at the same time, X' represents a chlorine atom;
to obtain a compound of the general formula Ib:

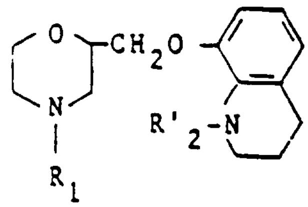

(Ib)

in which $R_1$ and $R'_2$ are as defined above.

**14.** The preparation process according to claim 13, characterised in that the reaction of compounds II and III is carried out in a solvent selected from the tertiary amides, at a temperature of from 90 to 110 ° C in the presence of an acceptor of the hydracid formed in the course of the reaction.

**15.** A process according to claim 14, characterised in that the acceptor is sodium hydride.

**16.** A process according to claim 13, characterised in that the hydrogenation of compound IV is carried out under low pressure in the presence of a catalyst belonging to the metals of group VIII, in a solvent such as the low − molecular − weight alcohols, at a temperature of from 20 to 60 ° C.

**17.** Process according to claim 16, characterised in that the hydrogenation is carried out under a hydrogen pressure of from $1 \times 10^5$ to $6 \times 10^5$ Pa, in the presence of Rh/C or $Pd(OH)_2/C$ as catalyst.

**18.** Process according to claim 13, characterised in that the condensation of compounds (Ia) and V is carried out in a solvent selected from the aromatic hydrocarbons or polar solvents, at a temperature of from 20 to 80 ° C in the presence of an acceptor of the hydracid formed.

**19.** Process according to claim 18, characterised in that the acceptor is a tertiary amine.

**20.** Process for the preparation of the compounds of claim 1 corresponding to the general formula I':

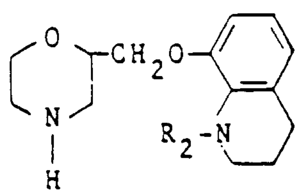

(I')

in which $R_2$ is as defined in claim 1, characterised in that a morpholine of the general formula II':

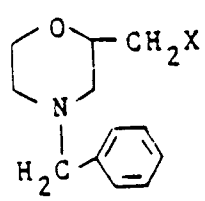

(II')

in which X is as defined in claim 13, is condensed with a hydroxyquinoline of the formula III:

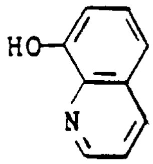

(III)

to obtain a compound of the formula IV':

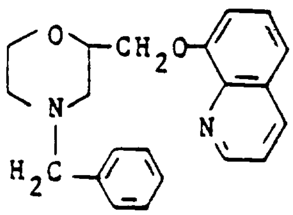

(IV')

which is subjected to catalytic hydrogenation under low pressure to obtain a compound of the formula IV":

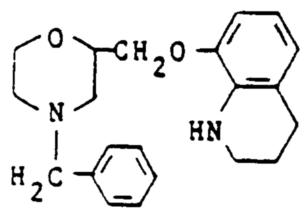

(IV")

which is: either debenzylated directly by hydrogenolysis to give the compound of the formula I'a:

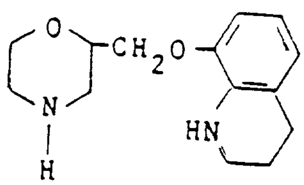

(I'a)

or is first alkylated or acylated by means of $R'_2X'$, which is as defined in claim 13, and then debenzylated by catalytic hydrogenation under low pressure to obtain the compound of the general formula I'b

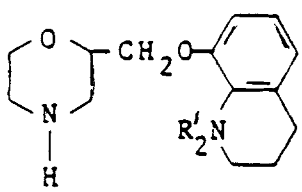

(I'b)

in which $R'_2$ is as defined in claim 13.

**21.** Pharmaceutical compositions containing as active ingredient a compound according to claims 1 to 12, with suitable pharmaceutical excipients.

**22.** Pharmaceutical compositions according to claim 21, presented in a form suitable especially for the treatment of ischaemic syndromes and cerebral ageing.

**Patentansprüche**

**1.** Morpholinderivate der allgemeinen Formel I

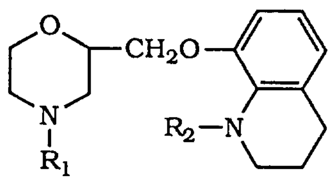

(I)

in der:
$R_1$ ein Wasserstoffatom;
eine geradkettige oder verzweigte Alkylgruppe mit 1 bis 6 Kohlenstoffatomen, die gegebenenfalls eine Doppelbindung enthält;
eine Aralkylgruppe der allgemeinen Formel:

$Ar-(CH_2)_m-$

in der:
Ar eine Arylgruppe, die gegebenenfalls durch eines oder mehrere Halogenatome, Alkylgruppen oder Alkoxygruppen mit jeweils 1 bis 5 Kohlenstoffatomen, oder Gruppen der Formel $-O-(CH_2)_n-O-$, in der n Werte von 1 oder 2 besitzt, substituiert ist; und
m eine ganze Zahl mit einem Wert von 1 bis 3 bedeuten;
eine Cycloalkylgruppe mit 5 oder 6 Kohlenstoffatomen; oder
eine Acylgruppe der Formel R'−CO−, worin R' eine Alkylgruppe mit 1 oder 2 Kohlenstoffatomen darstellt; und
$R_2$ ein Wasserstoffatom;
eine geradkettige oder verzweigte Alkylgruppe mit 1 bis 6 Kohlenstoffatomen, die gegebenenfalls eine Doppelbindung aufweist;
eine Aralkylgruppe der allgemeinen Formel: $Ar-(CH_2)_m-$, in der Ar und m die oben angegebenen Bedeutungen besitzen; oder
eine Acylgruppe der Formel R'−CO−, in der R' die oben angegebenen Bedeutungen besitzt; bedeuten, und
deren Enantiomeren.

**2.** Die physiologisch verträglichen Salze der Verbindungen gemäß Anspruch 1 mit geeigneten Säuren.

**3.** R,S−8−[(4−Benzyl−morpholin−2−yl)−methoxy]−1,2,3,4−tetrahydro−chinolin.

**4.** R,S−8−[(4−Cyclopentyl−morpholin−2−yl)−methoxy]−1,2,3,4−tetrahydrochinolin.

**5.** R,S−8−[(4−p−Chlorbenzyl−morpholin−2−yl)−methoxy]−1,2,3,4−tetrahydrochinolin.

**6.** R,S−8−[(Morpholin−2−yl)−methoxy]−1,2,3,4−tetrahydro−chinolin, dessen Dihydrochlorid und dessen Enantiomere.

**7.** R,S−1−Methyl−8−[(4−benzyl−morpholin−2−yl)−methoxy]−1,2,3,4−tetrahydro−chinolin.

**8.** R,S−1−Isopropyl−8−[(4−isopropyl−morpholin−2−yl)−methoxy]−1,2,3,4−tetrahydro−chinolin.

**9.** R,S−1−Acetyl−8−[[4−isopropyl−morpholin−2−yl)−methoxy]−1,2,3,4−tetrahydro−chinolin.

**10.** R,S−1−Acetyl−8−[(morpholin−2−yl)−methoxy]−1,2,3,4−tetrahydro−chinolin.

**11.** R,S – 8 – [(4 – Allyl – morpholin – 2 – yl) – methoxy] – 1,2,3,4 – tetrahydro – chinolin.

**12.** R,S – 8 – [(4 – Isopropyl – morpholin – 2 – yl) – methoxy] – 1,2,3,4 – tetrahydro – chinolin, dessen Dihydrochlorid und dessen Enantiomere.

**13.** Verfahren zur Herstellung der Verbindungen nach Anspruch 1, **dadurch gekennzeichnet,** daß man: ein substituiertes Morpholin der allgemeinen Formel II

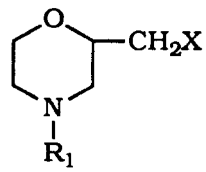

(II)

in der
$R_1$ die in Anspruch 1 angegebenen Bedeutungen besitzt und
X ein Halogenatom oder eine Tosyloxygruppe bedeutet,
mit Hydroxychinolin der Formel III

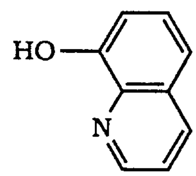

(III)

welches man zuvor in das Alkalisalz umgewandelt hat, umsetzt zur Bildung einer Verbindung der allgemeinen Formel IV

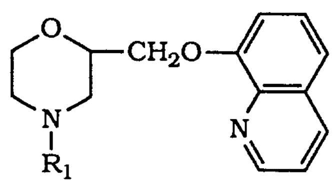

(IV)

in der $R_1$ die in Anspruch 1 angegebenen Bedeutungen besitzt;
die man anschließend hydriert zur Bildung eines Derivats der allgemeinen Formel Ia

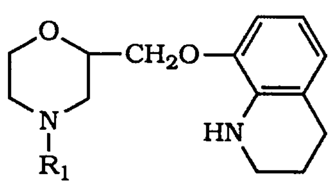

(Ia)

in der $R_1$ die in Anspruch 1 angegebenen Bedeutungen besitzt,
welches man dann mit einem Derivat der allgemeinen Formel V

$R'_2 - X'$ (V)

in der
$R'_2$ eine geradkettige oder verzweigte Alkylgruppe mit 1 bis 6 Kohlenstoffatomen, die gegebenenfalls eine Doppelbindung aufweist;
eine Aralkylgruppe der allgemeinen Formel: $Ar - (CH_2)_m -$, in der Ar und m die in Anspruch 1

angegebenen Bedeutungen besitzen; und
X' gleichzeitig ein Bromatom oder ein Iodatom oder eine Tosyloxygruppe bedeuten; oder
$R'_2$ eine Acylgruppe der Formel R' – CO –, in der R' die in Anspruch 1 angegebenen Bedeutungen besitzt, und gleichzeitig X' ein Chloratom bedeuten;
kondensiert zur Bildung eines Derivats der allgemeinen Formel Ib:

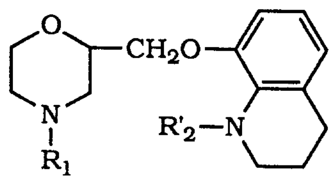

(Ib)

in der $R_1$ und $R'_2$ die oben angegebenen Bedeutungen besitzen.

**14.** Verfahren nach Anspruch 13, **dadurch gekennzeichnet,** daß man die Umsetzung der Derivate der Formeln II und III in einem aus tertiären Amiden ausgewählten Lösungsmittel bei einer Temperatur zwischen 90 und 110 ° C in Gegenwart eines Akzeptors für die im Verlaufe der Reaktion gebildete Wasserstoffsäure durchführt.

**15.** Verfahren nach Anspruch 14, **dadurch gekennzeichnet,** daß man als Akzeptor Natriumhydrid verwendet.

**16.** Verfahren nach Anspruch 13, **dadurch gekennzeichnet,** daß man die Hydrierung der Verbindung der Formel IV bei vermindertem Druck in Gegenwart eines aus Metallen der VIII. Gruppe des Periodensystems ausgewählten Katalysators in einem Lösungsmittel, wie einem niedrigmolekularen Alkohol, bei einer Temperatur zwischen 20 und 60 ° C bewirkt.

**17.** Verfahren nach Anspruch 16, **dadurch gekennzeichnet,** daß man die Hydrierung bei einem Wasserstoffdruck von 1 x $10^5$ bis 6 x $10^5$ Pa in Gegenwart von Rh/C oder $Pd(OH)_2/C$ als Katalysator durchführt.

**18.** Verfahren nach Anspruch 13, **dadurch gekennzeichnet,** daß man die Kondensation der Verbindungen der Formeln (Ia) und V in einem aus aromatischen Kohlenwasserstoffen oder polaren Lösungsmitteln ausgewählten Lösungsmittel bei einer Temperatur zwischen 20 und 80 ° C in Gegenwart eines Akzeptors für die gebildete Wasserstoffsäure durchführt.

**19.** Verfahren nach Anspruch 18, **dadurch gekennzeichnet,** daß man als Akzeptor ein tertiäres Amin verwendet.

**20.** Verfahren zur Herstellung der Verbindungen gemäß Anspruch 1 der allgemeinen Formel I':

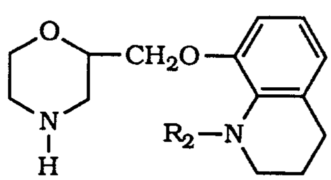

(I')

in der $R_2$ die in Anspruch 1 angegebenen Bedeutungen besitzt, **dadurch gekennzeichnet,** daß man ein Morpholin der allgemeinen Formel II':

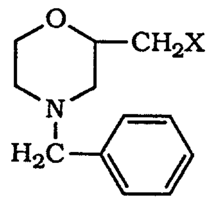

(II')

in der X die in Anspruch 13 angegebenen Bedeutungen besitzt, mit Hydroxychinolin der Formel III:

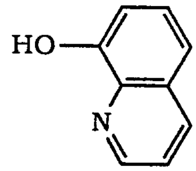

(III)

kondensiert zur Bildung eines Derivats der Formel IV':

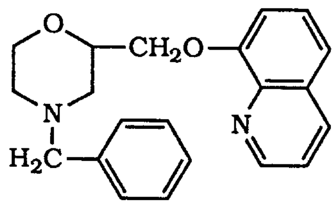

(IV')

welches man einer katalytischen Hydrierung bei vermindertem Druck unterwirft zur Bildung eines Derivats der Formel IV":

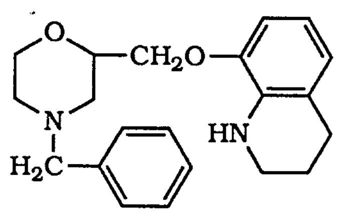

(IV")

welches man direkt durch Hydrogenolyse debenzyliert zur Bildung des Derivats der Formel I'a

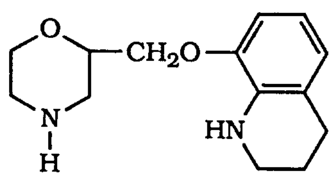

(I'a)

oder welches man zunächst mit der Verbindung der Formel $R'_2X'$, wie sie in Anspruch 13 definiert ist, alkyliert oder acyliert und dann durch katalytische Hydrierung bei vermindertem Druck debenzyliert zur Bildung des Derivats der Formel I'b

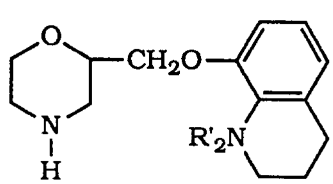

(I'b)

in der $R'_2$ die in Anspruch 13 angegebenen Bedeutungen besitzt.

**21.** Pharmazeutische Zubereitungen enthaltend als Wirkstoff eine Verbindung nach einem der Ansprüche 1 bis 12 in Kombination mit pharmazeutisch geeigneten Trägermaterialien.

**22.** Pharmazeutische Zubereitungen nach Anspruch 21 in einer insbesondere zur Behandlung von ischämischen Syndromen und Syndromen des Alterns des Gehirns geeigneten Form.